# EUROPEAN PATENT APPLICATION

(11) **EP 1 514 549 A1**
(43) Date of publication of application: **16.03.2005**
(21) Application number: 03733409.1
(22) Date of filing: 13.06.2003
(51) Int. Cl.: A61K 31/573, A61K 9/72, A61K 47/04, A61K 47/10, A61K 47/18, A61K 47/26, A61P 11/02, A61P 11/06, A61P 29/00, A61P 37/08

(54) **POWDERY RESPIRATORY TONIC COMPOSITION**

(30) Priority: 14.06.2002 JP 2002174402
(71) Applicant: SSP Co., Ltd., Chuo-ku, Tokyo 103-8481 (JP)
(72) Inventor: MIYADAI, Nobuo, NARITA-SHI, Chiba 286-0042 (JP); OKADA, Minoru, INZAI-SHI, Chiba 270-1323 (JP); HORIE, Toshiaki, KATORI-GUN, Chiba 287-0205 (JP)
(74) Representative: Hartz, Nikolai F., Dr.
(86) International application number: PCT/JP2003/007527
(87) International publication number: WO 2003/105859

(57) **Abstract**

The present invention relates to powdery inhalant compositions, each of which contains a steroidal anti-inflammatory drug, a carrier and water and has a water activity at 25°C of from 0.35 to 0.75.

Each powdery inhalant composition according to the present invention is high in the delivery rate of its steroidal anti-inflammatory drug to an inhalation target site from the nasal cavities or oral cavity, that is, to the alveoli, the bronchioles, the bronchial tubes or the airway, and allows the steroidal anti-inflammatory to exhibit its superb therapeutic effects.

## Description

### Technical Field

This invention relates to a powdery inhalant composition containing a steroidal anti-inflammatory drug useful for the prevention and treatment of inflammatory airway diseases such as bronchial asthma and rhinallergosis.

### Background Art

From recent researchs, airway diseases such as bronchial asthma and rhinallergosis (allergic rhinitis, vasomotor rhinitis, etc.) turned out to be chronic airway mucositis associated with various phlogocytes, such as mastocytes, eosinocytes and lymphocytes, and inflammatory diseases characterized by the aggravation of airway anaphylaxis based on such chronic airway mucositis. As a result, therapeutics conventionally used for these diseases, such as bronchodilators, antiallergics or antihistamics, are increasingly replaced by those containing so-called anti-inflammatory drugs equipped with effects such as the inhibition of a decrease of basophils or eosinocytes in the airway mucosa surface layer, a decrease of lymphocytes and a release of lymphokine from lymphocytes, the inhibition of a release of a mediator from basophils, the reduction of secretion from adenocytes, and the reduction of vasopermeability. Of these, the topotherapy with steroidal anti-inflammatory drugs has especially attracted a great interest for its significant therapeutic effects, and several inhalant steroidal preparations applicable to the airway have been developed to date.

Conventional inhalant medicaments are used by inhalation through the nasal cavities or oral cavity. However, due to the poor rates of their active ingredients reaching to target sites (the alveoli, bronchioles, bronchial tubes or airway), such medicaments have not been able to fully satisfy both effectiveness and safety, although they are designed for topical administration.

### Disclosure of the Invention

To develop inhalants which permit the efficient delivery of steroidal anti-inflammatory drugs as active ingredients to target sites, the present inventors have proceeded with various investigations. As a result, it has been found that a powdery preparation with a steroidal anti-inflammatory drug, a carrier and water added therein and with a water activity at 25°C controlled to 0.35 to 0.75 can selectively reach the alveoli, bronchioles, bronchial tubes or airway subsequent its inhalation through the nasal cavities or oral cavity, can effectively draw out the excellent therapeutic effects of the steroidal anti-inflammatory and can also reduce side effects, leading to the completion of the present invention.

Specifically, the present invention provides a powdery inhalant composition, which comprises a steroidal anti-inflammatory, a carrier and water and has a water activity at 25°C of from 0.35 to 0.75.

The present invention also provides a method of treatment of an inflammatory airway disease, which comprises intraoral inhalation or intranasal inhalation of the above-described powdery composition.

### Brief Description of the Drawings

FIG. 1 is a diagram of the water activity of powdery inhalant composition versus R_{f} value.
FIG. 2 is a diagram of employed lactose samples of different particle size distributions versus R_{f} value.

### Best Modes for Carrying out the Invention

In the powdery inhalant composition according to the present invention, the steroidal anti-inflammatory drug is a pharmaceutically-active ingredient, and no particular limitation is imposed thereon insofar as it is a steroidal compound having a pharmaceutical activity inducible by inhalation. Examples include compounds represented by the following formula (1) and their salts: 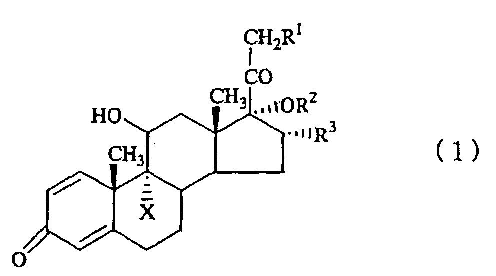 wherein R¹ represents a hydrogen atom, a halogen atom, a hydroxyl group or -OCOR⁴ in which R⁴ represents a linear or branched alkyl, cycloalkyl or aryl group which may be substituted by one or more halogen groups or cycloalkyl groups, R² represents a hydrogen atom, a lower alkanoyl group or a cycloalkanoyl group, R³ represents a hydrogen atom or a methyl group, and X represents a hydrogen atom or a halogen atom.

In the formula (1), examples of the halogen atoms represented by R¹ and X include fluorine, chlorine, bromine and iodine. Among these, chlorine or bromine is particularly preferred as R¹ while fluorine is especially preferred as X.

As the linear or branched alkyl group represented by R⁴, one having 1 to 23 carbon atoms, especially 1 to 15 carbon atoms is preferred. As the one or more halogen atoms which may substitute on such an alkyl group, fluorine, chlorine, bromine or iodine is preferred, with chlorine or bromine being particularly preferred. As the cycloalkyl group, one having 3 to 6 carbon atoms is preferred.

Preferred specific examples of the linear alkyl group represented by R⁴ include methyl, ethyl, n-propyl, n-butyl, n-nonyl, n-undecanyl, n-tridecanyl, and n-pentadecanyl. Preferred specific examples of the branched alkyl group include isopropyl, isobutyl, sec-butyl, t-butyl, isopentyl, neopentyl, t-pentyl, and isohexyl. Preferred specific examples of the halogenated alkyl group include 3-chloropropyl, 3-bromopropyl, 3-fluoropropyl, 4-chlorobutyl, 4-bromobutyl, 4-fluorobutyl, 5-chloropentyl, 5-bromopentyl, 5-fluoropentyl, 6-chlorohexyl, 6-bromohexyl, and 6-fluorohexyl. Preferred specific examples of the cycloalkylalkyl group include 2-cyclohexylethyl, 2-chylopropylethyl, 2-cyclopentylethyl, 3-cyclopropylpropyl, 3-cyclopentylpropyl, 3-cyclohexylpropyl, 4-cyclopropylbutyl, 4-cyclopentylbutyl, 4-cyclohexylbutyl, 5-cyclopropylpentyl, 5-cyclopentylpentyl, 5-cyclohexylpentyl, and 6-cyclopentylhexyl.

Preferred specific examples of the cycloalkyl group include cyclopropyl, cyclobutyl, cyclopentyl, and cyclohexyl.

Further, preferred specific examples of the aryl group include phenyl, naphthyl, 2-methylphenyl, 4-methylphenyl, 2-ethylphenyl, 4-ethylphenyl, 2-methoxyphenyl, 4-methoxyphenyl, 2-ethoxyphenyl, 4-ethoxyphenyl, 2-aminophenyl, 4-aminophenyl, 4-dimethylaminophenyl, 2-hydroxyphenyl, 4-hydroxyphenyl, 2-nitrophenyl, 4-nitrophenyl, 2-chlorophenyl, 4-chlorophenyl, 2-bromophenyl, 4-bromophenyl, 2-fluorophenyl, 4-fluorophenyl, 2,6-dichlorphenyl, 2,6-dibromophenyl, and biphenyl.

Of these, a hydroxyl group or cyclohexanecarbonyloxy group is particularly preferred as R¹ from the standpoint of effectiveness.

As the lower alkanoyl group represented by R², an alkanoyl group having 1 to 6 carbon atoms is preferred. Specific examples include acetyl, propionyl and butyryl. As the cycloalkanoyl group, one having 4 to 7 carbon atoms is preferred. Specific examples include cyclopropanecarbonyl, cyclobutanecarbonyl, cyclopentanecarbonyl, and cyclohexanecarbonyl.

As R², a cyclopropanecarbonyl group is particularly preferred. As R³, on the other hand, a methyl group is especially preferred.

Among the steroidal compounds of the formula (1), particularly preferred are 9-fluoro-11β,17,21-trihydroxy-16α-methyl-1,4-pregnadiene-3,20-dione 17-cyclopropanecarboxylate and 9-fluoro-11β,17,21-trihydroxy-16α-methyl-1,4-pregnadiene-3,20-dione 21-cyclohexanecarboxylate 17-cyclopropanecarboxylate. These steroidal compounds are described in JP-B-07116215.

The average particle size of the steroidal compound for use in the present invention may fall within a range of preferably from 0.3 to 20 µm, especially from 0.5 to 7 µm.

As the carrier for use in the present invention, no particular limitation is imposed thereon insofar as it is a powdery carrier, although a crystalline substance having high water solubility is preferred. Specifically, one or more carriers selected from saccharides, sugar alcohols, amino acids and inorganic salts are preferred.

Examples of the saccharides include glucose, lactose, sucrose, maltose, trehalose, and dextran. Examples of the sugar alcohols include mannitol, xylitol, erythritol, multitol, sorbitol, arabitol, dulcitol, paratinose, lactitol, inositol, and xylose. Examples of the amino acids include leucine, isoleucine, lysine, valine, threonine, methionine, cysteine, cystine, phenylalanine, tryptophan, and glycine. Examples of the inorganic salts include calcium carbonate, sodium chloride, and calcium phosphate. Among these, saccharides are preferred, with lactose being particularly preferred.

The average particle size and particle size distribution of such a carrier affect the efficiency of development of pharmaceutical activity. The average particle size can fall within a range of preferably from 1 to 500 µm, even preferably within a range of from 5 to 150 µm, especially preferably within a range of from 5 to 50 µm. As the particle size distribution, on the other hand, particles smaller than 50 µm in particle size can account preferably for 50% or more, especially 60% or more. More specifically, a particle size distribution of <10 µm: 7% or more, <20 µm: 12% or more and <50 µm: 50% or more is preferred, with a particle size distribution of <10 µm: 10% or more, <20 µm: 25% or more and <50 µm: 60% or more being especially preferred. As used herein, "particle size" refers to one measured by a laser diffraction/scattering analysis, and the average particle size is determined in terms of median diameter. The particle size distribution is expressed in terms of wt.%.

The powdery inhalant composition according to the present invention contains water. When the water activity at 25°C of the powdery inhalant composition is from 0.35 to 0.75, the delivery rate of the steroidal anti-inflammatory drug as the pharmaceutically-active ingredient to a target site is pronouncedly improved, and moreover, the long-term stability is good. Both with water activities lower than 0.35 and with water activities higher than 0.75, the delivery rate of the pharmaceutically-active ingredient to the target site is reduced.

Concerning the term "water activity" as used herein, P/P₀ is called "water activity (Aw)" in which P₀ refers to the vapor pressure of purified water at a predetermined temperature while P stands for the water vapor pressure of a sample. As water activity has a relationship with the relative humidity (RH) of air at equilibrium as expressed by RH = 100 × P/P₀, water activity can be expressed by the following formula: Aw = P/P₀ = RH%/100. When a sample is left over in a predetermined constant environment, absorption and desorption of water take place between the sample and the surrounding air. Once an equilibrium state is reached with the vapor pressure of the sample, the relative humidity (RH) of air at equilibrium remains constant. The Aw value, therefore, can be determined in accordance with the above formula. Specifically, when a sample is sealed in a container, the Aw of the sample can be determined by measuring the RH of the air inside the container.

To control the water activity, it is only necessary to decrease or increase the amount of free water on the surface of powder. As a method for lowering the water activity of powder, the powder can be dried in a box-type dryer, a fluidized bed or the like or can be left over in dry air. As a method for raising the water activity of powder, water can be added directly to the powder with a sprayer or the like (for example, water may be sprayed, for example, while feeding the powder in a fluidized bed) or the powder can be left over in air of high humidity.

For the effective exhibition of the pharmaceutical activity, the weight ratio of the steroidal anti-inflammatory drug to the carrier in the powdery inhalant composition according to the present invention may be preferably from 1:1,000 to 1:5, even preferably from 1:400 to 1:5, notably from 1:100 to 1:10.

In the powdery inhalant composition according to the present invention, pharmaceutically-active ingredients other than the steroidal anti-inflammatory can be incorporated. Examples thereof are antiallergics, antihistamics, antimicrobials, antifungals, diuretics, sympatholytics, sympathomimetics; sputum dissolvers, expectorants, cholinolytics, calcium antagonists, antivirals, non-steroidal anti-inflammatories, antipyretic analgesics, hormone agents, diabetes therapeutics, calcium metabolizers, anticancer drugs, and immunosuppressants.

The powdery inhalant composition according to the present invention can be produced preferably by mixing the above-described ingredients in a mixer, fluidized bed or the like such that the steroidal anti-inflammatory is dispersed in the carrier.

As sites to which the powdery inhalant composition according to the present invention can be administered, somatic cavities such as nasal cavities, oral cavities, airway, bronchial tubes and alveoli can be mentioned while they differ depending on the pharmaceutically-active ingredient. Administration can be effected by a spray pump or inhaler. Illustrative administration routes include the nasal route and oral route. For the administration through the nasal route or oral route, a dry powder inhaler, a dry powder spray device or the like can be used. These preparations include both single-dose preparations and multiple-dose preparations.

The effective amount of the steroidal anti-inflammatory in the powdery inhalant composition according to the present invention differs depending on the age, sex and disease severity of each patient. In general, however, it can be from 25 to 2,000 µg/day or so, preferably from 50 to 800 µg/day or so. The administration frequency for this daily dose may generally be from one to several times.

As diseases to be treated with the powdery inhalant composition according to the present invention, a variety of inflammatory respiratory diseases can be mentioned. As used herein, the term "inflammatory respiratory diseases" include, in addition to upper respiratory inflammation diseases and lower respiratory inflammation diseases, throat allergy, chronic obstructive lung diseases, and interstitial pneumonia. Examples of the upper respiratory inflammation diseases include rhinallergosis such as allergic rhinitis and vasomotor rhinitis (essential rhinitis), and sinusitis. Examples of the lower respiratory inflammation diseases include bronchitis, bronchial asthma, and infantile asthma. The term "allergic rhinitis" as used herein means any allergic response at the mycteric mucous membrane, and includes pollinosis (seasonal allergic rhinitis) and year-round allergic rhinitis, which are characterized by sneeze, nasal mucus, nasal congestion, itch, ocular itch, ocular redness and/or lacrimation. In terms of the morbidity of respiratory responses, bronchial asthma can be classified into immediate asthmatic response, late asthmatic response and post-late asthmatic response (allergic asthma) depending on the onset time of response. The powdery inhalant composition according to the present invention can be applied to the asthmatic response at any of these stages, and is effective especially for late asthmatic response which occurs several hours after exposure to an antigen and consists primarily of an inflammatory response of the airway.

### Examples

The present invention will next be described in detail based on examples.

### Production Example

Following the procedure described in JP-B-07116215, 9-fluoro-11β,17,21-trihydroxy-16α-methyl-1,4-pregnadiene-3,20-dione 17-cyclopropanecarboxylate (Compound 1) and 9-fluoro-11β,17,21-trihydroxy-16α-methyl-1,4-pregnadiene-3,20-dione 21-cyclohexanecarboxylate 17-cyclopropanecarboxylate (Compound 2) were synthesized.

Described specifically, a trialkyl ester of orthocyclopropanecarboxylic acid was reacted with dexamethasone in the presence of an acid to form an intramolecular orthoester derivative, which was then subjected to acid hydrolysis to afford Compound 1. A reactive derivative of cyclohexanecarboxylic acid was next reacted with Compound 1 to afford Compound 2. Using a grinding mill, each compound was ground into a powder of several micrometers.

### Example 1

In accordance with the following formulation, Compound 2 and lactose were mixed into a homogeneous dispersion to obtain an inhalation powder.

| | |
|---|---|
| Compound 2 | 0.2 mg |
| Lactose*⁾ | 4.8 mg |
| Total | 5.0 mg |

| | |
|---|---|
| *) Average particle size 31.1 µm | |

Particle size distribution
≤45 µm 57.6%
≤100 µm 88.2%
≤150 µm 92.4%
≤250 µm 98.3%

### Multi-stage liquid impinger testing method

A multi-stage liquid impinger is a testing apparatus shown as Apparatus 1 in United States Pharmacopoeia Vol. 24, and is operated basically following the procedure prescribed in United States Pharmacopoeia Vol. 24. Different from the procedure of United States Pharmacopoeia Vol. 24, however, the residual amounts and delivered amounts of 9 fractions of the compound in or on or to a capsule or blister pack, an inhaler, a mouthpiece adapter, an induction port, Stage 1, Stage 2, Stage 3, Stage 4 and Stage 5 (filter) were investigated (8 fractions where neither a capsule nor a blister pack existed or where one equivalent to a capsule was included in the inhaler), and "Rf value" was defined independently in the present invention. Usually, the percentage of the total of amounts of an active ingredient delivered to Stage 3, Stage 4 and Stage 5 based on an amount of the active ingredient emitted from an inhaler (fmitted dose) is called "respirable fraction (RF)", and this RF value may be referred to as "effective fine particle dose" or the like. In general, this RF value increases as the release of an active medicament from a carrier becomes easier. Accordingly, the inclusion of more free water in a powder is liable to an increase in the compatibility between its active ingredient and carrier, so that the release of the active ingredient from the carrier becomes difficult to result in a reduced RF value. Conversely, the inclusion of less free water in a powder leads to an increased RF value. With the inclusion of free water at an excessively low content, however, the influence of electrostatic charge or the like becomes greater, so that the emitted dose tends to become lower and the RF value increases accordingly. If the content of free water is extremely ether high or low, the total delivery rate to Stage 3, Stage 4 and Stage 5 will be forced decrease, so that no stable pharmaceutical activity can be expected. In the present invention, investigations were hence conducted by defining, as an RF value, the percentage of a total delivery rate to Stage 3, Stage 4 and Stage 5 based on a total delivery rate of the above-described 9 fractions (or 8 fractions).

As it is prescribed to conduct a test by lowering the internal pressure of the testing apparatus to a negative pressure of 4.0 kpa, the inhalation flow rate (L/min) in a test differs from inhaler to inhaler (because the draw resistance differs depending on the inhaler). When the inhalation flow rate varies in a range of from 30 to 60 L/min, for example, the cutoff value of Stage 2 varies in a range of from 9.6 to 6.8 µm. Therefore, the test was conducted using inhalers of the same inhalation flow rate. Specifically, each inhaler employed in the test had an inhalation flow rate of 45 L/min and the cutoff value of Stage 2 was 7.9 µm. This means that particles of the compound smaller than 7.9 µm in average particle size reached Stage 3, Stage 4 or Stage 5. It is to be noted that to control of the water activities of samples for use in this test, lactose samples of different water activities were obtained by lowering the water activity of lactose stepwise in a box-type dryer, spraying water to the lactose with a sprayer to provide lactose samples of predetermined water activities, and then mixing the lactose samples in a mixer.

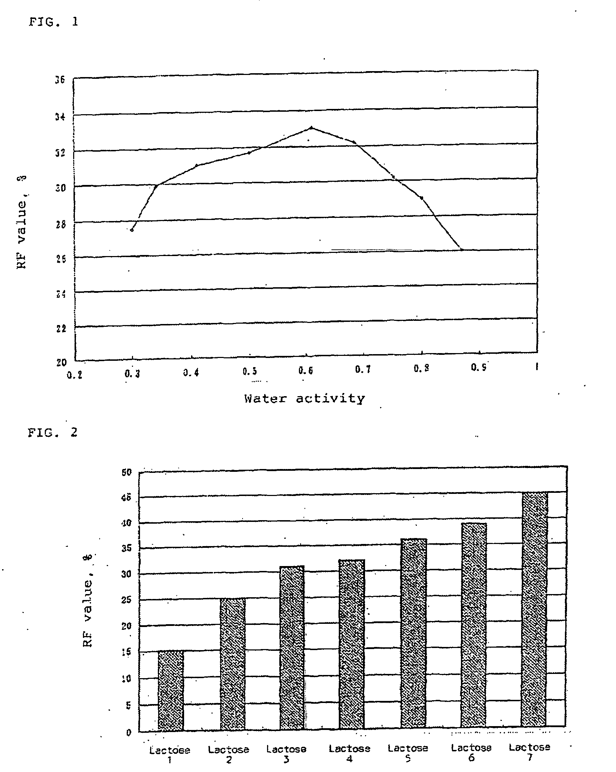

The results are presented in FIG. 1. As clearly envisaged from FIG. 1, the powdery inhalant with the steroidal anti-inflammatory contained therein significantly differed in R_{f} value depending on its water activity. To obtain an R_{f} value of 30% or greater, the water activity is required to fall within a range of from 0.35 to 0.75 so that this range is preferred.

### Example 2

In accordance with the formulation of Example 1, inhalation powders were formulated by using lactose samples different in particle size distribution. Using those powders, R_{f} values were measured in a similar manner as in Example 1.

The particle size distributions of the employed lactose samples are presented in Table 1, and the R_{f} values of the inhalation powders making use of the lactose samples are presented in FIG. 2.

As evident from Table 1 and FIG. 2, it is appreciated that the use of lactose having a particle size distribution centered on a smaller particle size, especially a particle size distribution containing 50% or more, notably 60% or more particles smaller than 50 µm leads to a particularly high R_{f} value.

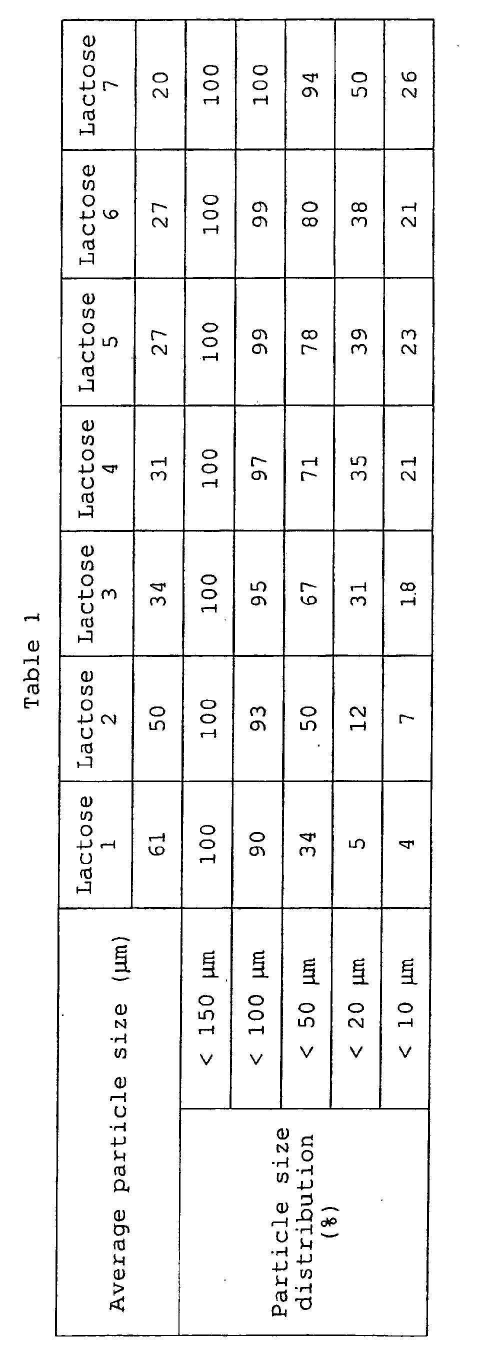

### Industrial Applicability

Each powdery inhalant composition according to the present invention is high in the delivery rate of its steroidal anti-inflammatory drug to an inhalation target site from the nasal cavities or oral cavity, that is, to the alveoli, the bronchioles, the bronchial tubes or the airway, and allows the steroidal anti-inflammatory to exhibit its superb therapeutic effects.

## Claims

1. A powdery inhalant composition comprising a steroidal anti-inflammatory drug, a carrier and water and having a water activity at 25°C of from 0.35 to 0.75.

2. A powdery inhalant composition according to claim 1, wherein said steroidal anti-inflammatory drug is a compound represented by the following formula (1) or a salt thereof: 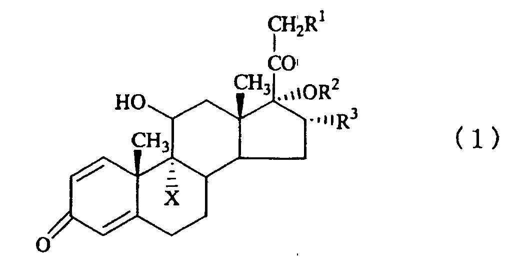 wherein R¹ represents a hydrogen atom, a halogen atom, a hydroxyl group or -OCOR⁴ in which R⁴ represents a linear or branched alkyl, cycloalkyl or aryl group which may be substituted by one or more halogen groups or cycloalkyl groups, R² represents a hydrogen atom, a lower alkanoyl group or a cycloalkanoyl group, R³ represents a hydrogen atom or a methyl group, and X represents a hydrogen atom or a halogen atom.

3. A powdery inhalant composition according to claim 2, wherein in said formula (1), R¹ is a cyclohexanecarbonyloxy group, R² is a cyclopropanecarbonyl group, R³ is a methyl group, and X is a fluorine atom.

4. A powdery inhalant composition according to any one of claims 1-3, wherein said carrier comprises one or more carriers selected from saccharides, sugar alcohols, amino acids and inorganic salts.

5. A powdery inhalant composition according to any one of claims 1-4, wherein said carrier has a particle size distribution containing 50% or more particles smaller than 50 µm in particle size.

6. A powdery inhalant composition according to any one of claims 1-5, wherein a weight ratio of said steroidal anti-inflammatory drug to said carrier is from 1:100 to 1:10.

7. A powdery inhalant composition according to any one of claims 1-5, which is an intraoral inhalant preparation or an intranasal inhalant preparation.

8. A method of treatment of an inflammatory airway disease, which comprises intraoral inhalation or intranasal inhalation of a powdery composition as defined in claim 1.
